# EUROPEAN PATENT APPLICATION

(11) **EP 2 745 765 A1**
(43) Date of publication of application: **25.06.2014**
(21) Application number: 13806121.3
(22) Date of filing: 01.05.2013
(51) Int. Cl.: A61B 1/00, G02B 23/24

(54) **INSERTION DEVICE**

(30) Priority: 21.06.2012 JP 2012139939
(71) Applicant: Olympus Medical Systems Corp., Tokyo 151-0072 (JP)
(72) Inventor: OKAMOTO, Yasuhiro, Shibuya-ku Tokyo 151-0072 (JP)
(74) Representative: Schicker, Silvia
(86) International application number: PCT/JP2013/062711
(87) International publication number: WO 2013/190923

(57) **Abstract**

A pulling wire 10, a joystick 8, a pulley 21 capable of providing a pull assisting force to the pulling wire 10 by rotation, a C-shaped ring 22 that contacts the pulley 21 with a friction force with an input operation of the joystick 8 and thereby transmits the rotation of the pulley 21 to the pulling wire 10 are provided, and at the C-shaped ring 22, there is provided an end portion 22it of a cutout portion K that removes wear debris that is scraped from the pulley 21 with contact of the C-shaped ring 22 with the pulley 21 from the pulley 21.

## Description

### Technical Field

The present invention relates to an insertion apparatus including an insertion portion that is inserted into a subject, wherein the insertion portion is provided with an action portion.

### Background Art

In recent years, insertion apparatuses having insertion portions that are inserted into subjects, for example, endoscopes have been widely used in a medical field and an industrial field.

An endoscope that is used in the medical field enables observation of an organ in a body cavity by an elongated insertion portion being inserted into the body cavity that is a subject, and enables various kinds of treatments with use of a treatment instrument inserted into an insertion channel for the treatment instrument that the endoscope includes, in accordance with necessity.

Further, an endoscope that is used in the industrial field enables inspection such as observation of flaws, corrosion and the like of sites to be examined in an object, and various kinds of treatments, by an elongated insertion portion of the endoscope being inserted into the inside of the object such as the inside of a jet engine and piping of a factory.

Here, a configuration is known, in which an action portion, for example, a bending portion that is bendable in a plurality of directions is provided at a distal end side in an insertion direction of the insertion portion of an endoscope (hereinafter, simply called a distal end side).

More specifically, a distal end (hereinafter, simply called a distal end) in the insertion direction of a pulling wire which is a pulling member that is inserted through the insides of the insertion portion and the operation portion is connected to the bending portion, and the pulling wire is operated to be pulled by an operation member that is provided at the operation portion, whereby the bending portion is bendable in a plurality of directions.

Here, the configuration of an endoscope having an electrically assisting mechanism that performs assistance in the pulling operation of the bending wire by electric power is also well known, and is disclosed in Patent Literature 1, for example.

The electrically assisting mechanism disclosed in Japanese Patent Application Laid-Open Publication No. 2009-005836 has the configuration that has the pulley which is a drive force generating member that rotates in one direction by a motor or the like, and the C-shaped ring that is a drive force assisting member that is provided on an outer periphery of the pulley to be contactable to the pulley with a frictional force, in the operation portion, wherein the intermediate position of the pulling wire is wound around the C-shaped ring.

The electrically assisting mechanism has such a configuration that when the pulling wire is pulled by the operation member, the C-shaped ring is reduced in diameter by the pulling wire that is wound around the C-shaped ring, contacts the pulley with a frictional force, and rotates in one direction with the pulley, whereby a pull assisting force is given to the pulling wire by the rotation.

However, in the configuration disclosed in Japanese Patent Application Laid-Open Publication No. 2009-005836, when the C-shaped ring contacts the outer periphery of the pulley with a frictional force, the member configured by a material with a lower modulus of elasticity, namely, a softer material, when the pulley and the C-shaped ring are compared, is scraped.

Note that it is found out that debris that is scraped from a soft member (hereinafter, called wear debris) easily adheres to a soft member side. Namely, when the C-shaped ring is softer than the pulley, wear debris easily adheres to the C-shaped ring on one hand. On the other hand, when the pulley is softer than the C-shaped ring, wear debris easily adheres to the pulley. It is considered that this is because a softer member has higher adhesiveness.

Thus, the wear debris adheres to the member formed by a softer material when comparing the pulley and the C-shaped ring and if the wear debris enters between the outer periphery of the pulley and the C-shaped ring, when the C-shaped ring is brought into contact with the outer periphery of the pulley with a frictional force, vibration or abnormal noise is caused by the wear debris. Further, there has been a problem that the above-mentioned pull assisting force is not made constant and unstable.

Note that the foregoing problems concern not only the pulley and the C-shaped ring as disclosed in Japanese Patent Application Laid-Open Publication No. 2009-005836, but also a drive force generating member and a drive force assisting member. Further, the problems are not limited to the endoscope but may also arise with respect to the other insertion apparatuses.

The present invention has been made in view of the above problems and an object of the present invention is to provide an insertion apparatus having a configuration capable of removing wear debris, which is generated with a contact of a drive force assisting member with a drive force generating member and adheres to a member having a lower modulus of elasticity out of the drive force generating member and the drive force assisting member, easily and simply.

### Disclosure of Invention

### Means for Solving the Problem

An insertion apparatus according to one aspect of the present invention includes an insertion portion to be inserted into a subject and in which an action portion is provided at the insertion portion. The insertion apparatus comprises: a pulling member that is inserted through the insertion portion, has a distal end in an insertion direction of the insertion portion fixed to the action portion, and causes the action portion to act by pulling; an operation member that is provided at an operation portion at a proximal end in the insertion direction of the insertion portion for performing an input of a pulling operation of the pulling member; a drive force generating member that is provided in the operation portion and capable of providing a pull assisting force to the pulling member by rotation; a drive force assisting member that is provided in the operation portion and contacts the drive force generating member with a friction force with an input operation of the operation member and thereby transmits the rotation of the drive force generating member to the pulling member; and a removal portion that is provided at any one member having a higher modulus of elasticity out of the drive force generating member and the drive force assisting member, and removes wear debris that is scraped from the other member having a low modulus of elasticity with contact of the drive force assisting member to the drive force generating member from the other member.

### Brief Description of the Drawings

Fig. 1 is a perspective view schematically showing an endoscope showing a first embodiment;
Fig. 2 is a view schematically showing only a configuration that causes a bending portion of Fig. 1 to bend;
Fig. 3 is a view schematically showing a state in which a joystick of Fig. 2 is operated to tilt, and the bending portion is bent;
Fig. 4 is a plan view showing a state in which wear debris adhering to an outer periphery of a pulley is removed by a cutout portion of a C-shaped ring of Fig. 2 under enlargement;
Fig. 5 is a plan view showing a modification in which a cleaning blade is provided at an end portion in one end of the cutout portion of the C-shaped ring of Fig. 2 under enlargement;
Fig. 6 is a sectional view showing a modification in which an outer layer portion of the pulley of Fig. 2 is formed to be softer than an inner layer portion, with the C-shaped ring under enlargement;
Fig. 7 is a plan view showing a modification in which a concave portion is formed on the outer periphery of the pulley of Fig. 2, with the C-shaped ring under enlargement;
Fig. 8 is a plan view showing a modification of the shape of the C-shaped ring of Fig. 2, with the pulley under enlargement;
Fig. 9 is a perspective view showing a modification of a shape of the C-shaped ring of Fig. 2 different from that of Fig. 8;
Fig. 10 is a plan view of the C-shaped ring of Fig. 9 seen from an X direction in Fig. 9 with the pulley under enlargement;
Fig. 11 is a view schematically showing only a configuration that causes a bending portion of an insertion portion of an endoscope of a second embodiment to bend;
Fig. 12 is a view schematically showing a state in which a joystick in Fig. 11 is operated to tilt, and the bending portion is bent;
Fig. 13 is a plan view showing a modification in which a cleaning blade is provided at an end face of a pressed member of Fig. 11, and a concave portion is provided on an outer periphery of a pulley under enlargement;
Fig. 14 is a view schematically showing only a configuration that causes a bending portion of an insertion portion of an endoscope of a third embodiment to bend;
Fig. 15 is a view of the configuration that causes the bending portion to bend of Fig. 14 seen from a XV direction in Fig. 14;
Fig. 16 is a view showing a section along a XVI-XVI line in Fig. 15, with a bottom surface of a pulley under enlargement;
Fig. 17 is a plan view showing a modification in which a concave groove is not formed on the bottom surface of the pulley of Fig. 15, but a concave groove is formed in a surface of a friction plate that contacts the pulley, under enlargement; and
Fig. 18 is a side view showing a modification in which a cleaning blade is provided in the concave groove of fig. 17, from a XVII direction in Fig. 17.

### Best Mode for Carrying Out the Invention

Hereinafter, embodiments of the present invention will be described with reference to the drawings. Note that in the embodiments shown as follows, an insertion apparatus will be described with an endoscope cited as an example.

### (First Embodiment)

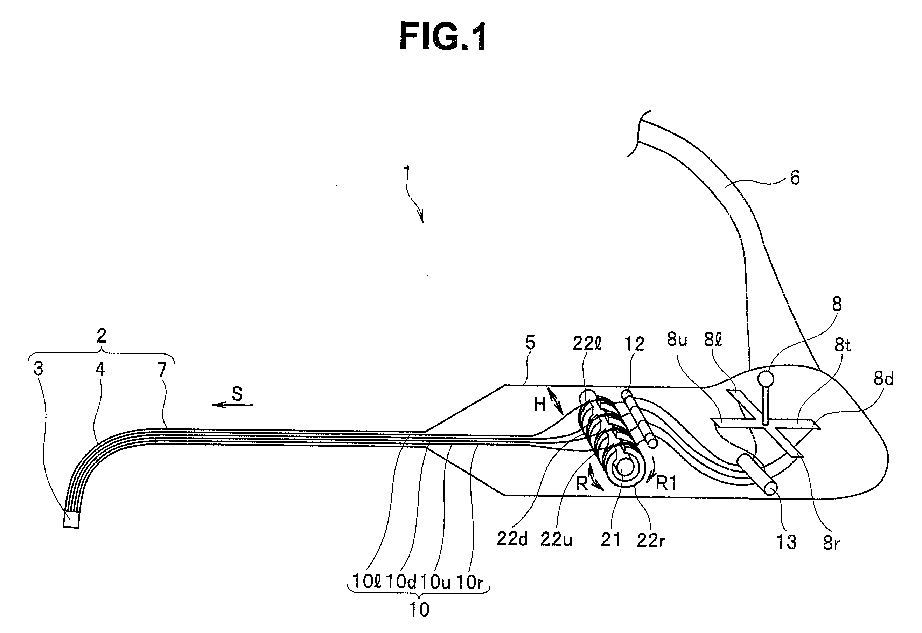

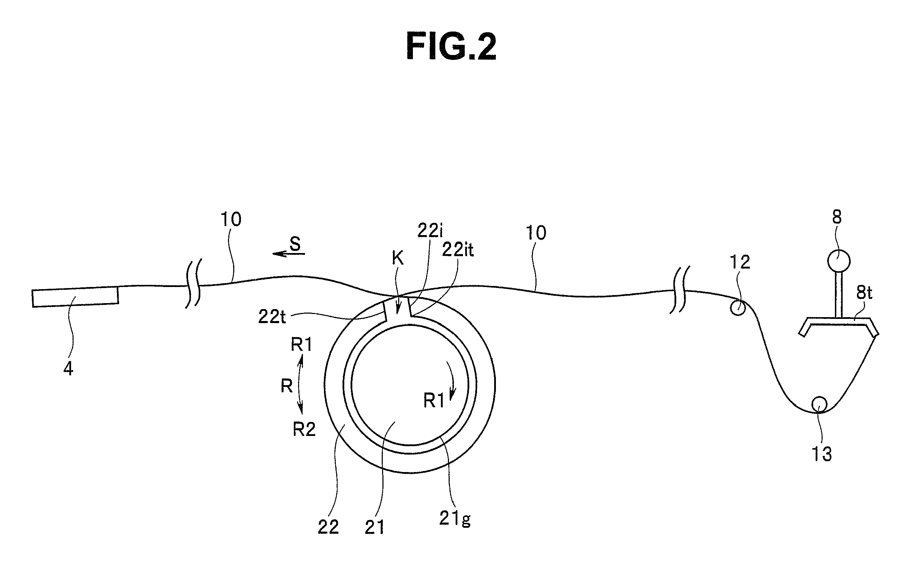

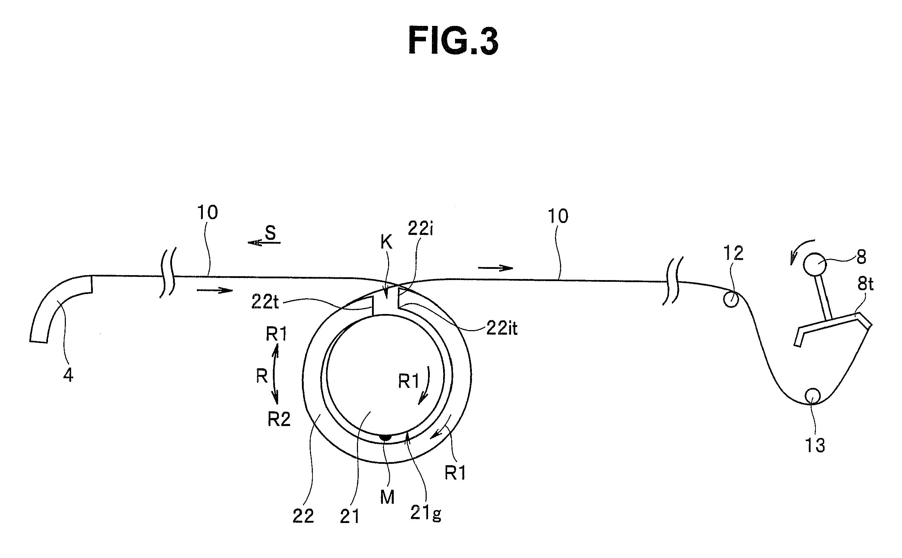

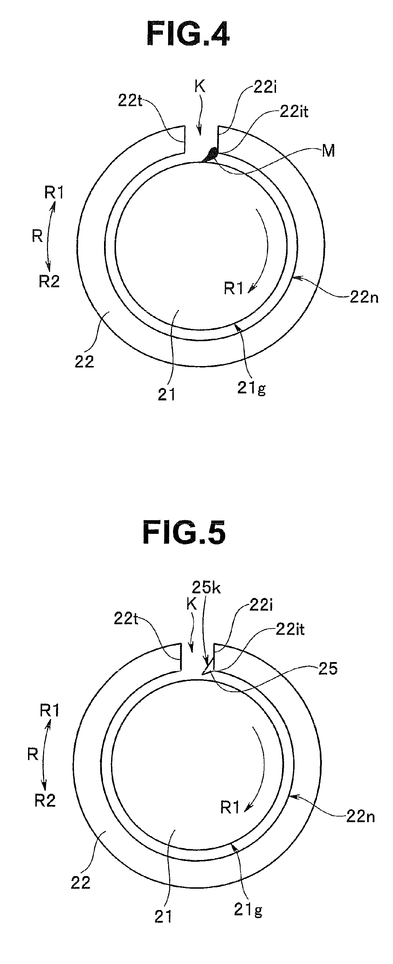

Fig. 1 is a perspective view schematically showing an endoscope showing the present embodiment. Fig. 2 is a view schematically showing only a configuration that causes a bending portion of Fig. 1 to bend. Fig. 3 is a view schematically showing a state in which a joystick of Fig. 2 is operated to tilt, and the bending portion is bent. Fig. 4 is a plan view showing a state in which wear debris adhering to an outer periphery of a pulley is removed by a cutout portion of a C-shaped ring of Fig. 2 under enlargement.

As shown in Fig. 1, an endoscope 1 has a main part configured by including an insertion portion 2 that is inserted into a subject, an operation portion 5 that is connected to a proximal end in an insertion direction S of the insertion portion 2 (hereinafter, simply called a proximal end), and a universal cord 6 that is extended from the operation portion 5. Further, the endoscope 1 is connectable to a peripheral apparatus via a connector not illustrated that is provided at an extension end of the universal cord 6.

The insertion portion 2 has a main part configured by a distal end portion 3, a bending portion 4 that is an action portion, and a flexible tube portion 7, and is formed to be elongated along the insertion direction S.

The bending portion 4 is operated to bend in a plurality of directions, for example, four directions of up, down, left and right by, for example, a joystick 8 that is an operation member provided at the operation portion 5. Note that the joystick 8 performs input of a pulling operation of a pulling wire 10 that will be described later.

Briefly describing a bending configuration of the bending portion 4, pulling wires 10r, 101, 10d and 10u (hereinafter, collectively called a pulling wire 10) that are a plurality of, for example, four pulling members are respectively inserted through insides of the insertion portion 2 and the operation portion 5 by being displaced by substantially 90° in a circumferential direction of the insertion portion 2.

Distal ends of the respective pulling wires 10r, 101, 10d and 10u are connected to the bending portion 4, and proximal ends are respectively fixed to respective end portions 8r, 81, 8d and 8u of a suspension frame 8t with a shape in plan view being cruciform, of the joystick 8. Note that what is described as the pulling wire 10 is applied to all of the pulling wires 10r, 101, 10d and 10u.

The pulling wire 10r causes the bending portion 4 to bend in a right direction by being pulled with a tilting operation that is an input operation to the right direction by the joystick 8.

Further, the pulling wire 101 causes the bending portion 4 to bend in a left direction by being pulled with a tilting operation that is an input operation to the left direction by the joystick 8.

Further, the pulling wire 10d causes the bending portion 4 to bend in a down direction by being pulled with a tilting operation that is an input operation to the down direction by the joystick 8.

The pulling wire 10u causes the bending portion 4 to bend in an up direction by being pulled with a tilting operation that is an input operation to the up direction by the joystick 8.

Inside the operation portion 5, a pulley 21 that is a drive force generating member that is capable of giving a pull assisting force to the pulling wire 10 by rotation, and a C-shaped ring 22 that is a drive force assisting member that transmits the rotation of the pulley 21 to the pulling wire 10 by contacting the pulley with a frictional force with the input operation of the joystick 8 are provided.

The pulley 21 is formed into a columnar shape elongated along a width direction H of the operation portion 5 that is a direction orthogonal to the insertion direction S, and is configured to rotate in one direction R1 of rotational directions R by a motor not illustrated at all times when a power supply of the endoscope 1 is on.

As the C-shaped ring 22, four C-shaped rings 22 are provided as C-shaped rings 22r, 221, 22d and 22u respectively spaced from one another along the width direction H in an outer periphery 21g (see Fig. 2) of the pulley 21, are located to be contactable to the outer periphery 21g of the pulley 21, and are each formed into a C-shape by being partially cut out. Note that what is described as the C-shaped ring 22 will be applied to all of the C-shaped rings 22r, 221, 22d and 22u hereinafter.

Further, the C-shaped ring 22 is formed into a shape without an outer diameter difference in a radial direction along the rotational direction R, as shown in Fig. 2.

Note that in the present embodiment, the C-shaped ring 22 is configured by a material with a higher modulus of elasticity than the pulley 21. Namely, the pulley 21 is formed to be softer than the C-shaped ring 22. Consequently, the C-shaped ring 22 configures one member in the present embodiment, whereas the pulley 21 configures the other member in the present embodiment.

Further, around outer peripheries of the respective four C-shaped rings 22r, 221, 22d and 22u, intermediate positions of the pulling wires 10r, 101, 10d and 10u are respectively wound.

More specifically, as shown in Fig. 2, the pulling wire 10 that is extended to a rear side in the insertion direction S (hereinafter, simply called a rear side) from the bending portion 4 is wound around the outer periphery of the C-shaped ring 22 by being wound from one end 22i side in a cutout portion K (see Fig. 2) that is formed at the C-shaped ring 22 to be along the outer periphery of the C-shaped ring 22, and thereafter being extended to the rear side from the other end 22t side.

Thereby, a distal end side of the pulling wire 10 is configured to be pulled when the C-shaped ring 22 is rotated in the one direction R1 and slackened when the C-shaped ring 22 is rotated in the other direction R2 at an opposite side from the one direction R1.

Further, the distal end sides of the pulling wires 10r, 10l, 10d and 10u that are located between the C-shaped ring 22 and the bending portion 4 are located slack when the joystick 8 is not operated, as shown in Fig. 2. Further, proximal end sides in the insertion direction S of the pulling wires 10r, 101, 10d and 10u that are located between the C-shaped ring 22 and the joystick 8 (hereinafter, simply called the proximal end sides) are also located slack via guide rollers 12 and 13 when the joystick 8 is not operated, and respective proximal ends are respectively connected to the respective end portions 8r, 81, 8d and 8u of the suspension frame 8t of the joystick 8.

As shown in Fig. 3, the C-shaped ring 22 is reduced in diameter, and contacts the pulley 21 with a frictional force as the pulling wire 10 is pulled by the input operation of the joystick 8, and transmits the pull assisting force from the pulley 21 to the pulling wire 10 by rotating in the one direction R1 with the pulley 21.

More specifically, when the pulling wire 10u is pulled, as the joystick 8 is tilted in the up direction, for example, the C-shaped ring 22u is reduced in diameter, and contacts the outer periphery 21 g of the pulley 21 with a frictional force.

Thereby, the C-shaped ring 22u transmits the pull assisting force from the pulley 21 to the distal end side of the pulling wire 10u by rotating in the one direction R1 with the pulley 21. As a result, the distal end side of the pulling wire 10u is pulled, whereby the bending portion 4 bends in the up direction.

Note that at this time, the C-shaped rings 22r, 221 and 22d are not in contact with the outer periphery 21g of the pulley 21, and therefore, the pulling wires 10r, 101 and 10d are not pulled. However, when the joystick 8 is tilted in a position between the up direction and the right direction, the C-shaped ring 22r as well as the C-shaped ring 22u contacts the outer periphery 21 g of the pulley 21, whereby the pulling wire 10u and the pulling wire 10r are pulled at the same time. Namely, when the joystick 8 is titled in the middle directions of up, down, left and right, two of the C-shaped rings contact the outer periphery 21g.

Further, the above pulling operation similarly applies to the C-shaped rings 22r, 221 and 22d. More specifically, the bending portion 4 is configured to be bent in any one of the right, the left and the down directions by using the fact that when the pulling wires 10r, 101 and 10d are respectively pulled as the joystick 8 is tilted in the right, the left and the down directions respectively, the C-shaped rings 22r, 221 and 22d are respectively reduced in diameter, and respectively contact the outer periphery 21 g of the pulley 21 with frictional forces.

Note that as described above, the C-shaped ring 22 rotates in the one direction R1 with the pulley 21 after the C-shaped ring 22 is reduced in diameter, but does not rotate integrally with the pulley 21. Namely, the C-shaped ring 22 rotates in the one direction R1 in a state in which the rotational speed differs from that of the pulley 21. In other words, the C-shaped ring 22 rotates in the one direction R1 while sliding with respect to the outer periphery 21 g of the pulley 21.

Further, as shown in Fig. 4, in the present embodiment, an end portion 22it that faces the outer periphery 21 g of the pulley 21 in the one end 22i of the cutout portion K of the C-shaped ring 22 configures a removal portion that removes wear debris M that is scraped from the pulley 21 and adheres to the outer periphery 21 g of the pulley 21 by the C-shaped ring 22 contacting the outer periphery 21 g of the pulley 21, from the pulley 21.

Next, an operation of the present embodiment will be described.

When the joystick 8 is not operated, the C-shaped ring 22 is not in contact with the outer periphery 21 g of the pulley 21 that rotates in the one direction R1 by the motor, as shown in Fig. 2.

In order to cause the bending portion 4 to bend by using the aforementioned configuration in the above state, the joystick 8 is tilted in, for example, the up direction first as shown in Fig. 3, whereby the position that is slackened between the C-shaped ring 22u and the joystick 8, in the pulling wire 10u is pulled.

Thereby, as described above, the C-shaped ring 22u is reduced in diameter, whereby the C-shaped ring 22u contacts the outer periphery 21 g of the pulley 21 that rotates in the one direction R1 with a frictional force.

As a result, the C-shaped ring 22u also rotates in the one direction R1 with the pulley 21, and thereby the pull assisting force from the pulley 21 is transmitted to the position that is slackened between the bending portion 4 and the C-shaped ring 22u, in the pulling wire 10u, whereby the position is pulled. Thereby, the bending portion 4 to which the distal end of the pulling wire 10u is fixed bends in the up direction.

Note that when the input operation of the joystick 8 is released, the position between the C-shaped ring 22u and the joystick 8 in the pulling wire 10u is slackened, whereby the C-shaped ring 22u separates from the outer periphery 21 g of the pulley 21.

As a result, the C-shaped ring 22u individually rotates in the other direction R2, whereby the position between the bending portion 4 and the C-shaped ring 22u in the pulling wire 10u is slackened, as a result of which, the bending portion 4 returns to an unbending state.

Note that the above operation similarly applies to the occasion in which the bending portion 4 is caused to bend in the down direction, the right direction and the left direction.

Here, when the C-shaped ring 22 contacts the outer periphery 21 g of the pulley 21 with a frictional force, the outer periphery 21 g of the pulley 21 is scraped by the C-shaped ring 22, with the contact, because the pulley 21 is formed to be softer than the C-shaped ring 22 as described above.

Note that the wear debris M that is scraped adheres to the outer periphery 21g of the pulley 21 that is softer than the C-shaped ring 22 as shown in Fig. 3, for the aforementioned reason. As shown in Fig. 4, when the wear debris M that adheres to the outer periphery 21g of the pulley 21 reaches the cutout portion K of the C-shaped ring 22 with rotation in the one direction R1, the wear debris M is peeled and removed to an outside of a space between the outer periphery 21 g of the pulley 21 and an inner circumferential face 22n of the C-shaped ring 22, from the outer periphery 21 g of the pulley 21 by the end portion 22it of the C-shaped ring 22 in the one end 22i of the cutout portion K.

Note that removal of the wear debris M in the end portion 22it can be performed in the state in which the C-shaped ring 22 is in contact with the outer periphery 21g of the pulley 21, and in the case in which the C-shaped ring 22 rotates in the one direction R1 while the C-shaped ring 22 is in contact with the outer periphery 21 g of the pulley 21 and sliding.

As above, it is shown that the pulley 21 is formed to be softer than the C-shaped ring 22 in the present embodiment. Further, it is shown that the wear debris M that is scraped from the pulley 21 and adheres to the outer periphery 21 g of the pulley 21, with contact of the C-shaped ring 22 to the outer periphery 21 g of the pulley 21, is peeled and removed to an outside of the space between the outer periphery 21 g of the pulley 21 and the inner circumferential face 22n of the C-shaped ring 22 by the end portion 22it in the one end 22i of the cutout portion K of the C-shaped ring 22.

According to the above, the wear debris M does not enter the space between the outer periphery 21 g of the pulley 21 and the inner circumferential face 22n of the C-shaped ring 22. Thereby, when the C-shaped ring 22 contacts the outer periphery 21 g of the pulley 21 with a frictional force, it does not happen that the aforementioned pull assisting force does not become constant and becomes unstable, in addition to that vibration and abnormal sound occur due to the wear debris M.

From the above, the endoscope 1 can be provided, which has the configuration that can easily and simply remove the wear debris M that is generated with contact of the C-shaped ring 22 to the pulley 21, and adheres to the pulley 21.

Note that hereinafter, a modification will be shown with use of Fig. 5. Fig. 5 is a plan view showing the modification in which a cleaning blade is provided at the end portion in the one end of the cutout portion of the C-shaped ring of Fig. 2 under enlargement.

In the aforementioned present embodiment, it is shown that the wear debris M that is scraped from the pulley 21 and adheres to the outer periphery 21 g of the pulley 21 with contact of the C-shaped ring 22 to the outer periphery 21 g of the pulley 21 is peeled and removed to the outside of the space between the outer periphery 21 g of the pulley 21 and the inner circumferential face 22n of the C-shaped ring 22 from the outer periphery 21 g of the pulley 21 by the end portion 22it in the one end 22i of the cutout portion K of the C-shaped ring 22.

The present invention is not limited to the above, and as shown in Fig. 5, a configuration may be adopted, that peels and removes the wear debris M to the outside of the space between the outer periphery 21 g of the pulley 21 and the inner circumferential face 22n of the C-shaped ring 22 from the outer periphery 21 g of the pulley 21 by using a cleaning blade 25 in a blade shape that is provided to face the outer periphery 21 g of the pulley 21 and has an inclined surface 25k in the one end 22i.

Note that the cleaning blade 25 may be formed integrally with the C-shaped ring 22, or may be formed to be a separate piece.

Note that in the configuration as above, the cleaning blade 25 configures the removal portion in the present embodiment.

In the configuration shown in Fig. 5 as above, the wear debris M can be positively removed from the outer periphery 21g of the pulley 21 with use of the cleaning blade 25, and therefore, removability of the wear debris M is enhanced more than that of the aforementioned present embodiment. Note that the other effect is similar to that of the aforementioned present embodiment.

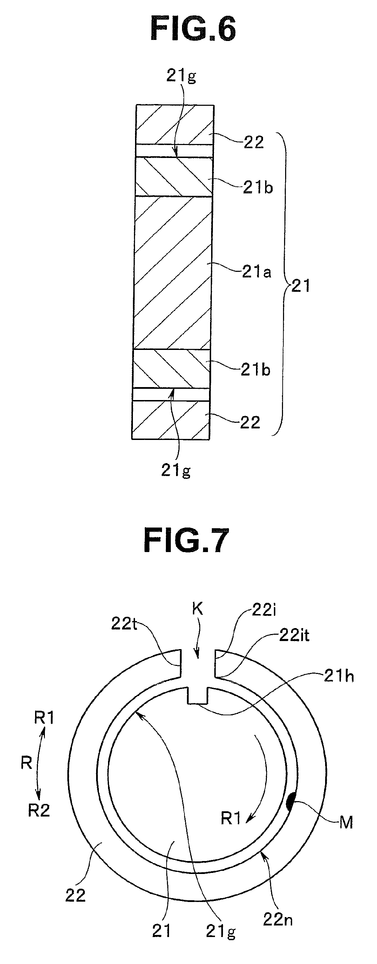

Further, hereinafter, a modification will be shown with use of Fig. 6. Fig. 6 is a sectional view showing the modification in which an outer layer portion of the pulley of Fig. 2 is formed to be softer than an inner layer portion, with the C-shaped ring under enlargement.

As shown in Fig. 6, the pulley 21 is configured by an inner layer portion 21a, and an outer layer portion 21b that is provided at an outer side of the inner layer portion 21 a, and the outer layer portion 21 b is configured by a material with a lower modulus of elasticity than the inner layer portion 21a, namely, the outer layer portion 21b may be formed to be softer than the inner layer portion 21a. Note that as a matter of course, the outer layer portion 21 b is formed to be softer than the C-shaped ring 22.

According to the configuration as above, even when the inner layer portion 21a is formed to be rigid in order to enhance rigidity of the pulley 21, if the outer layer portion 21 b is formed to be softer than the inner layer portion 21 a and the C-shaped ring 22, the wear debris M that is scraped from the outer layer portion 21b can be caused to adhere to the outer periphery 21 g of the outer layer portion 21 b similarly to the aforementioned present embodiment. Further, adhesion of the C-shaped ring 22 to the outer layer portion 21b can be enhanced. Note that the other effect is similar to that of the aforementioned present embodiment.

Further, Fig. 6 shows the example of the case of the pulley 21 being formed by the two layers, but the pulley 21 may be formed by three layers or more. Note that in this case, the outermost layer also needs to be formed to be softer than the inner layer portion 21a and the C-shaped ring 22.

Further, hereinafter, a modification will be shown with use of Fig. 7. Fig. 7 is a plan view showing the modification in which a concave portion is formed on the outer periphery of the pulley of Fig. 2, with the C-shaped ring under enlargement.

In the aforementioned present embodiment, the C-shaped ring 22 is configured by a material with a higher modulus of elasticity than the pulley 21. Namely, it is shown that the pulley 21 is formed to be softer than the C-shaped ring 22.

The pulley 21 is not limited to the above, and the pulley 21 is configured by a material with a higher modulus of elasticity than the C-shaped ring 22, totally contrarily to the present embodiment. Namely, the C-shaped ring 22 may be formed to be softer than the pulley 21. In this case, the pulley 21 configures the one member, whereas the C-shaped ring 22 configures the other member.

In the configuration as above, with contact of the C-shaped ring 22 to the outer periphery 21g of the pulley 21, the wear debris M that is scraped from the C-shaped ring 22 adheres to the C-shaped ring 22 that is softer than the pulley 21 as described above, but the wear debris M that adheres to the inner circumferential face 22n of the C-shaped ring 22 is removed by a concave portion 21h that is formed in the outer periphery 21 g of the pulley 21 as shown in Fig. 7.

Therefore, in the configuration in which the C-shaped ring 22 is formed to be softer than the pulley 21, the concave portion 21h configures the removal portion that removes the wear debris M that is scraped from the C-shaped ring 22 and adheres to the inner circumferential face 22n of the C-shaped ring 22 by the C-shaped ring 22 contacting the outer periphery 21 g of the pulley 21, from the C-shaped ring 22.

In the configuration as above, the endoscope 1 can be provided, which has the configuration that can easily and simply remove the wear debris M that is generated with contact of the C-shaped ring 22 to the pulley 21, and adheres to the C-shaped ring 22.

Note that in the present configuration, the C-shaped ring 22 may be configured by the two layers or more this time as shown in Fig. 6, and an outermost layer of the C-shaped ring 22 may be formed to be softer than the pulley 21 and an inner layer portion of the C-shaped ring 22.

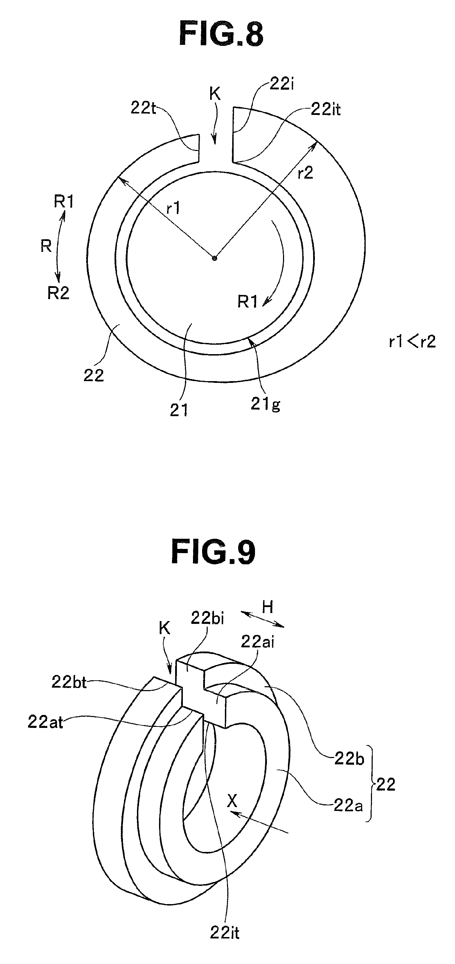

Further, hereinafter, a modification will be shown with use of Fig. 8. Fig. 8 is a plan view showing the modification of the shape of the C-shaped ring of Fig. 2, with the pulley under enlargement.

In the aforementioned present embodiment, it is shown that the C-shaped ring 22 is formed into a shape without an outer diameter difference in the radial direction along the rotational direction R, as shown in Fig. 2.

The C-shaped ring 22 is not limited to the above, and as shown in Fig. 8, the C-shaped ring 22 may be formed into a shape partially having an outer diameter difference along the rotational directions R, for example, for example, a shape in which a diameter r2 at the one end 22i side of the cutout portion K is formed to be a larger diameter than a diameter r1 at the other end 22t side (r2>r1).

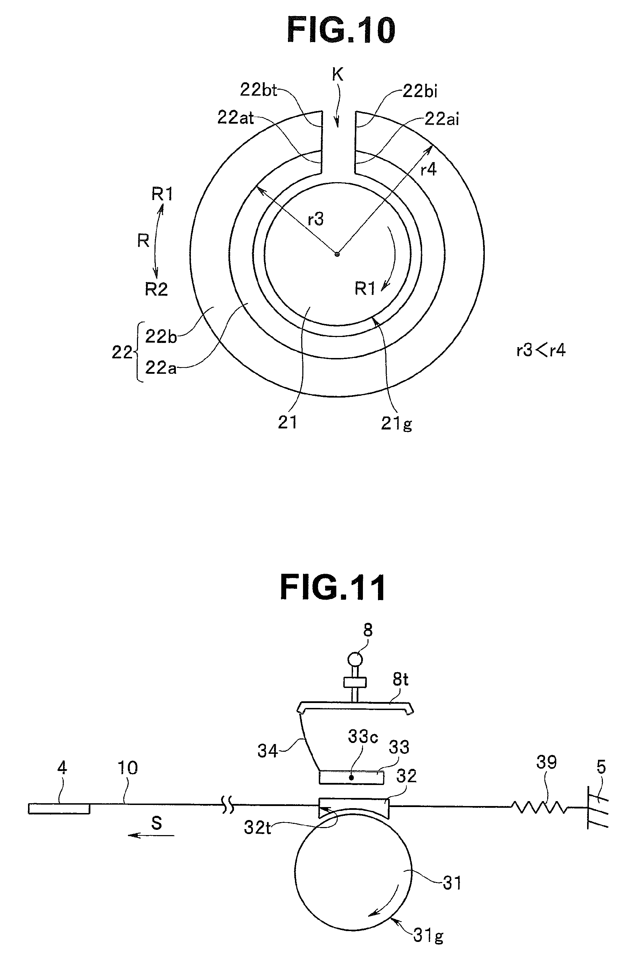

Furthermore, hereinafter, another modification will be shown with use of Fig. 9 and Fig. 10. Fig. 9 is a perspective view showing the modification in a different shape from that of Fig. 8 of the C-shaped ring of Fig. 2. Fig. 10 is a plan view showing the C-shaped ring of Fig. 9 seen from an X direction in Fig. 9 with the pulley, under enlargement.

Further, as shown in Fig. 9 and Fig. 10, the C-shaped ring 22 may be formed into a shape in which a C-shaped ring 22a that has a diameter r3 and is formed into a shape without an outer diameter difference in a radial direction along the rotational direction R, and a C-shaped ring 22b that has a diameter r4 that is larger than the diameter r3 (r3<r4) and is formed into a shape without an outer diameter difference in a radial direction along the rotational direction R are coaxially pasted on each other in the width direction H.

In the C-shaped ring 22 that has the shape as above, the pulling wire 10 that is extended to the rear side from the bending portion 4 is wound around the outer periphery of the C-shaped ring 22 by being wound from one end 22bi side in the cutout portion K that is formed in the C-shaped ring 22, partially running along an outer periphery of the C-shaped ring 22b, thereafter, partially running along an outer periphery of the C-shaped ring 22a, and thereafter, being extended to the rear side from the other end 22at side of the C-shaped ring 22a.

### (Second embodiment)

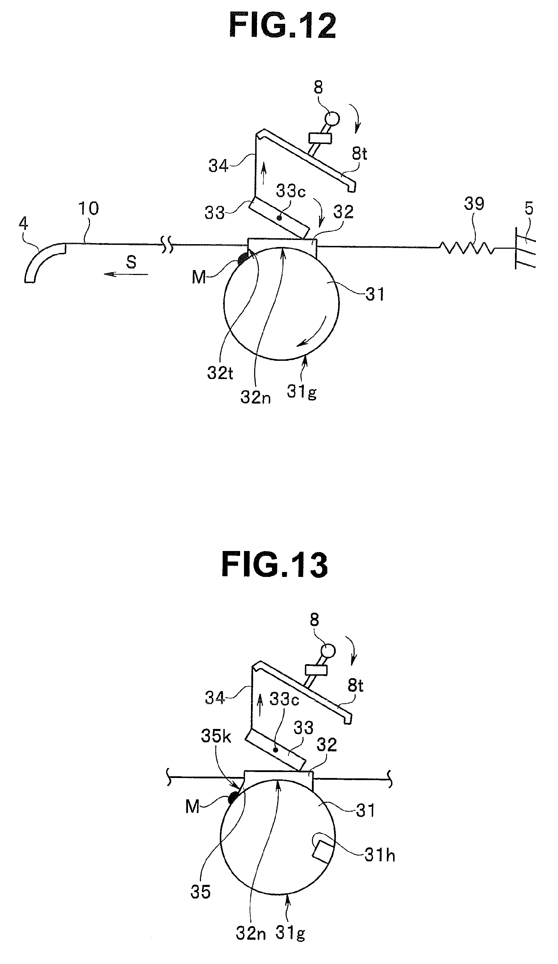

Fig. 11 is a view schematic showing only a configuration that causes a bending portion of an insertion portion of an endoscope of the present embodiment to bend. Fig. 12 is a view schematically showing a state in which a joystick of Fig. 11 is operated to tilt, and the bending portion is caused to bend.

A configuration of the endoscope of the second embodiment differs in that the C-shaped ring is not used for the drive force assisting member, but a pressing member that is rotated by an operation member, and a pressed member contactable to a pulley by pressing of the pressing member are used, as compared with the endoscope of the first embodiment shown in Fig. 1 to Fig. 4 described above.

Therefore, only the difference will be described, and the similar components as those of the first embodiment are assigned with the same reference signs, and the explanation thereof will be omitted.,

Note that in the present embodiment, what is described as the pulling wire 10 are also similarly applied to all of the pulling wires 10r, 101, 10d and 10u.

As shown in Fig. 11, in the present embodiment, a proximal end of the pulling wire 10 is fixed to an inner wall of the operation portion 5 via a spring 39 for removing slack of the pulling wire 10.

Further, in the present embodiment, a drive force assisting member that is provided in the operation portion 5, and transmits rotation of a pulley 31 to the pulling wire 10 by contacting, with a frictional force, the pulley 31 that rotates in the one direction R1 that is a drive force generating member with input operation of the joystick 8 is configured by a pressing member 33 and a pressed member 32. Note that the pressing member 33 is a member rotatable with a support point 33c as a center via a wire 34 by the input operation of the joystick 8. Further, the pressed member 32 is a member that has an intermediate position in the insertion direction S of the pulling wire 10 locked thereto, and contacts an outer periphery 31g of the pulley 31 with a frictional force by pressing by rotation of the pressing member 33.

Note that the pulley 31 has a configuration similar to that of the pulley 21 of the aforementioned first embodiment. Therefore, the pulley 31 may be configured by two layers or more as shown in Fig. 6.

As shown in Fig. 12, the pressed member 32 has a function of transmitting a pull assisting force from the pulley 31 to the pulling wire 10 by rotating in the one direction R1 with the pulley 31 by contacting the outer periphery 31 g of the pulley 31 with a frictional force. Note that the pressed member 32 does not rotate integrally with the pulley 31, but rotates while sliding with respect to the outer periphery 31 g of the pulley 31.

Four of the pressed members 32 are provided as pressed members 32r, 321, 32d and 32u (none of them is illustrated) by being respectively separated from one another along the width direction H in the outer periphery 31g of the pulley 31, and are located to be contactable to the outer periphery 31 g of the pulley 31.

Note that, in ordinary cases, for example, when the joystick 8 is tilted in the up direction, only the pressed member 32u contacts the outer periphery 31 g. However, when the joystick 8 is tilted to a position between the up direction and the right direction, the pressed member 32r also contacts the outer periphery 31g, similarly to the aforementioned first embodiment. Namely, when the joystick 8 is tilted in the intermediate directions of up, down, left and right, two of the pressed members contact the outer periphery 31 g.

Further, in the operation portion 5, four of the pressing members 33 are provided as pressing members 33r, 331, 33d and 33u (none of them is illustrated) respectively to face the respective pressed members 32r, 321, 32d and 32u.

Wires 34r, 341, 34d and 34u (none of them is illustrated) are extended respectively from the respective pressing members 33r, 331, 33d and 33u, and extension ends of the respective wires 34 are respectively connected to the respective end portions 8r, 81, 8d and 8u of the suspension frame 8t of the joystick 8.

Note that hereinafter, what is described as the pressed member 32 will be applied to all of the pressed members 32r, 321, 32d and 32u, and what is described as the pressing member 33 will be applied to all of the pressing members 33r, 331, 33d and 33u. What is described as the wire 34 will be applied to all of the wires 34r, 341, 34d and 34u.

Further, in the present embodiment, the pressed member 32 is configured by a material with a higher modulus of elasticity than the pulley 31. Namely, the pulley 31 is formed to be softer than the pressed member 32. Thereby, the pressed member 32 configures one member in the present embodiment, and the pulley 31 configures the other member in the present embodiment.

Further, in the present embodiment, an end face 32t of the pressed member 32 configures a removal portion that removes the wear debris M that is scraped from the pulley 31 and adheres to the outer periphery 31g of the pulley 31 by the pressed member 32 contacting the outer periphery 31 g of the pulley 31.

Next, an operation of the present embodiment will be described.

When the joystick 8 is not operated, the pressed member 32 is not in contact with the outer periphery 31g of the pulley 31 that rotates in the one direction R1 by a motor, as shown in Fig. 11.

In order to cause the bending portion 4 to bend by using the aforementioned configuration in the above state, the joystick 8 is tilted, for example, in the up direction first, as shown in Fig. 12, whereby the pressing member 33u rotates in one direction with the support point 33c as the center via the wire 34u.

Thereafter, the pressed member 32u contacts the outer periphery 31g of the pulley 31 with a frictional force by being pressed by the pressing member 33u.

The pressed member 32u rotates in the one direction R1 with the pulley 31 after the pressed member 32u contacts the outer periphery 31 g of the pulley 31, whereby the pull assisting force from the pulley 31 is transmitted to the pulling wire 10u, and thereby a position thereof is pulled. Thereby, the bending portion 4 to which the distal end of the pulling wire 10u is fixed bends in the up direction.

Note that when the input operation of the joystick 8 is released, the pressing member 33u rotates in the other direction via the wire 34u, whereby the pressed member 32u separates from the outer periphery 31 g of the pulley 31. As a result, the pressed member 32u individually rotates in the other direction R2, whereby the pulling wire 10u is slackened, and therefore, the bending portion 4 returns to an unbending state.

Note that the above similarly applies to the occasion in which the bending portion 4 is caused to bend in the down direction, the right direction and the left direction.

Here, when the pressed member 32 contacts the outer periphery 31g of the pulley 31 with a frictional force, the outer periphery 31g of the pulley 31 is scraped by the pressed member 32 with contact, because the pulley 31 is formed to be softer than the pressed member 32, as described above.

Note that the scraped wear debris M adheres to the outer periphery 31 g of the pulley 31 that is softer than the pressed member 32 as shown in Fig. 12, for the aforementioned reason. When the wear debris M that adheres to the outer periphery 31g of the pulley 31 reaches the pressed member 32 with rotation in the one direction R1, the wear debris M is peeled and removed to an outside of a space between the outer periphery 31g of the pulley 31 and an inner circumferential face 32n from the outer periphery 31 g of the pulley 31 by the end face 32t having an end portion of the inner circumferential face 32n that faces the outer periphery 31g of the pulley 31, of the pressed member 32.

Note that removal of the wear debris M in the pressed member 32 can be performed in the state in which the pressed member 32 is in contact with the outer periphery 31 g of the pulley 31, and in the case in which the pressed member 32 rotates in the one direction R1 while the pressed member 32 is in contact with the outer periphery 31g of the pulley 31 and sliding.

As above, in the present embodiment, it is shown that the pulley 31 is formed to be softer than the pressed member 32. Further, the wear debris M that is scraped from the pulley 31 and adheres to the outer periphery 31 g of the pulley 31 with contact of the pressed member 32 to the outer periphery 31g of the pulley 31 is peeled and removed to the outside of the space between the outer periphery 31g of the pulley 31 and the inner circumferential face 32n of the pressed member 32 from the outer periphery 31g of the pulley 31, by the end face 32t of the pressed member 32.

According to the above, the wear debris M does not enter the space between the outer periphery 31g of the pulley 31 and the inner circumferential face 32n of the pressed member 32, and therefore, it does not happen that when the pressed member 32 contacts the outer periphery 31 g of the pulley 31 with a frictional force, the aforementioned pull assisting force does not become constant and becomes unstable in addition to that vibration and unusual sound occur due to the wear debris M.

From the above, the endoscope 1 can be provided, which has the configuration that can easily and simply remove the wear debris M that is generated with contact of the pressed member 32 to the pulley 31, and adheres to the pulley 31.

Note that hereinafter, a modification will be shown with use of Fig. 13. Fig. 13 is a plan view showing the modification in which a cleaning blade is provided at the end face of the pressed member of Fig. 11 and a concave portion is provided in the outer periphery of the pulley under enlargement.

In the aforementioned present embodiment, it is shown that the wear debris M that is scraped from the pulley 31 and adheres to the outer periphery 31g of the pulley 31 with contact of the pressed member 32 to the outer periphery 31 g of the pulley 31 is peeled and removed to the outside of the space between the outer periphery 31 g of the pulley 31 and the inner circumferential face 32n of the pressed member 32, from the outer periphery 31g of the pulley 31 by the end face 32t of the pressed member 32.

The configuration of removal is not limited to the above, and may be a configuration that peels and removes the wear debris M to the outside of the space between the outer periphery 31g of the pulley 31 and the inner circumferential face 32n, from the outer periphery 31 g of the pulley 31, by using a cleaning blade 35 in a blade shape that is provided to face the outer periphery 31 g of the pulley 31 in the end face 32t of the pressed member 32, and has an inclined surface 35k, as shown in Fig. 13.

Note that the cleaning blade 35 may be formed integrally with the pressed member 32, or may be formed to be a separate piece.

Note that in the configuration as above, the cleaning blade 35 configures the removal portion in the present embodiment.

In the configuration shown in Fig. 13 as above, the wear debris M can be positively removed from the outer periphery 31 g of the pulley 31 with use of the cleaning blade 35, and therefore removability of the wear debris M is enhanced more than in the aforementioned present embodiment. Note that the other effect is similar to that of the aforementioned present embodiment.

Further, in the aforementioned present embodiment, the pressed member 32 is configured by the material with a higher modulus of elasticity than the pulley 31. Namely, it is shown that the pulley 31 is formed to be softer than the pressed member 32.

Totally contrarily to the present embodiment, the pulley 31 is configured by a material with a higher modulus of elasticity than the pressed member 32, without being limited to the above. Namely, the pressed member 32 may be formed to be softer than the pulley 31. In this case, the pulley 31 configures the one member, and the pressed member 32 configures the other member.

In the configuration as above, the wear debris M that is generated by the pressed member 32 being scraped adheres to the pressed member 32 that is softer than the pulley 31 as described above, with contact of the pressed member 32 to the outer periphery 31 g of the pulley 31, but the wear debris M that adheres to the inner circumferential face 32n of the pressed member 32 is removed by a concave portion 31h that is formed in the outer periphery 31 g of the pulley 31, as shown in Fig. 13.

Therefore, in the configuration in which the pressed member 32 is formed to be softer than the pulley 31, the concave portion 31h configures a removal portion that removes the wear debris M that is scraped from the pressed member 32 and adheres to the inner circumferential face 32n by the pressed member 32 contacting the outer periphery 31g of the pulley 31, from the pressed member 32.

In the configuration as above, the endoscope 1 can be provided, which has the configuration that can easily and simply remove the wear debris M that is generated with contact of the pressed member 32 to the pulley 31 and adheres to the pressed member 32 can be provided.

Note that in the present configuration, the pressed member 32 also may be configured by two layers or more this time as shown in Fig. 6, and an outermost layer of the pressed member 32 may be formed to be softer than the pulley 31 and an inner layer portion of the pressed member 32.

Further, Fig. 13 shows the case in which the concave portion 31h is formed in the outer periphery 31 g of the pulley 31, and the case in which the cleaning blade 35 is formed at the pressed member 32 together for convenience of explanation, but the concave portion 31h and the cleaning blade 35 are usually provided separately.

### (Third embodiment)

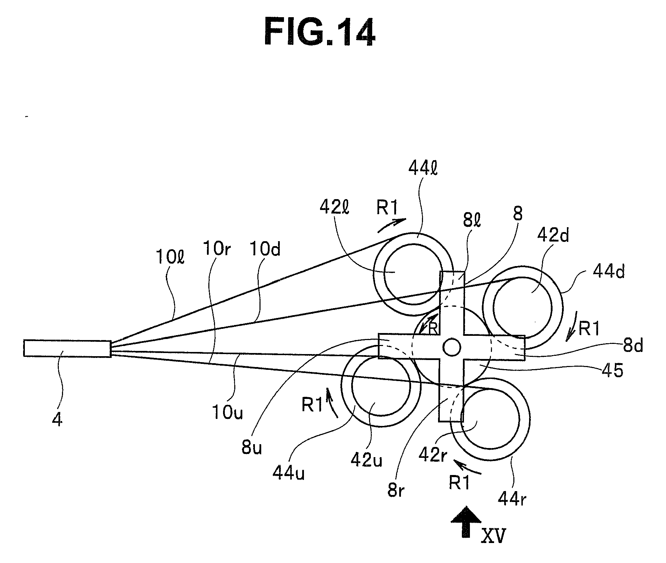

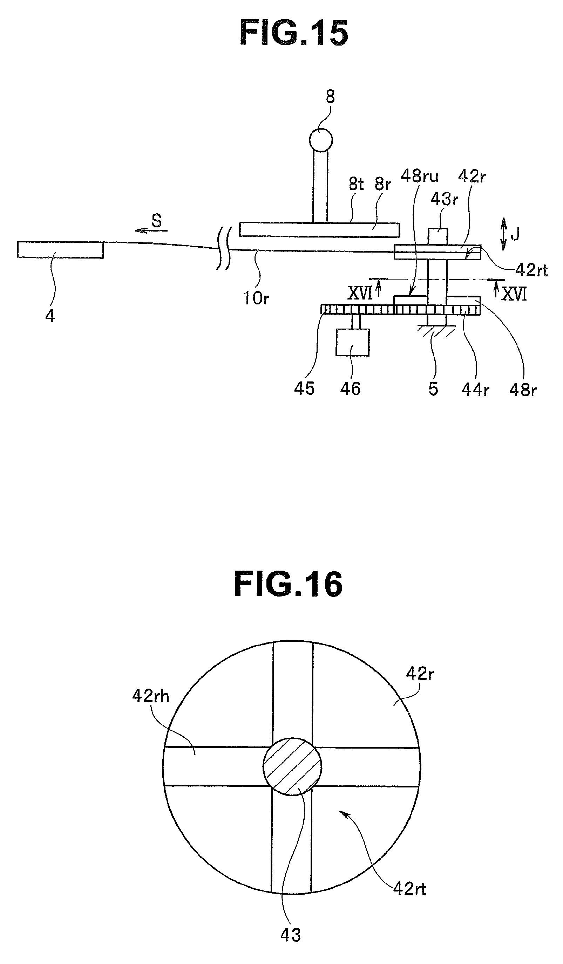

Fig. 14 is a view schematically showing only a configuration that causes a bending portion of an insertion portion of an endoscope of the present embodiment to bend. Fig. 15 is a view of the configuration that causes the bending portion to bend of Fig. 14 seen from a XV direction in Fig. 14. Fig. 16 is a view showing a section along a XVI-XVI line in Fig. 15, with a bottom surface of a pulley under enlargement.

A configuration of the endoscope of the third embodiment differs in that a drive force generating member is configured by a friction plate, and in that a drive force assisting member is configured by a pulley, as compared with the endoscope of the first embodiment shown in Fig. 1 to Fig. 4 described above, and the endoscope of the second embodiment shown in Fig. 11 and Fig. 12.

Therefore, only the differences will be described, the components similar to those in the first and the second embodiments are assigned with the same reference signs, and the explanation thereof will be omitted.

As shown in Fig. 14 and Fig. 15, in the operation portion 5, a pulley 42 (42r, 421, 42u and 42d) that are rotatable drive force assisting members that are pivotally supported movably in an axial direction J with respect to respective shafts 43r, 431, 43u and 43d, and a friction plate 48 (48r, 481, 48u and 48d, the friction plates 481, 48u and 48d are not illustrated) that are drive force generating members that are pivotally supported with respect to a shaft 43 (43r, 431, 43u and 43d) and are provided at an inner wall side of the operation portion 5 from the respective pulleys 42r, 421, 42u and 42d, and rotate in the one direction R1 are provided. Further, in the operation portion 5, a gear 44 (44r, 441, 44u and 44d (the gears 441, 44u and 44d are not illustrated)) that rotate in the one direction R1 with the respective friction plates 48r, 481, 48u and 48d that are provided at the inner wall side of the operation portion 5 from the respective pulleys 42r, 421, 42u and 42d with respect to the respective shafts 43r, 431, 43u and 43d, a gear 45 that is meshed with the respective gears 44r, 441, 44u and 44d and rotates in the one direction R1, and a motor 46 that causes the gear 45 to rotate in the one direction R1 are provided.

Note that hereinafter, what is described as the shaft 43 will be applied to all of the shafts 43r, 431, 43d and 43u, and what is described as the pulley 42 will be applied to all of the pulleys 42r, 421, 42d and 42u. Further, what is described as the friction plate 48 will be applied to all of the friction plates 48r, 481, 48d and 48u, and what is described as the gear 44 will be applied to all of the gears 44r, 441, 44d and 44u. Further, what is described as the pulling wire 10 will be applied to all of the pulling wires 10r, 101, 10d and 10u.

Note that with respect to the shaft 43, the pulley 42, the friction plate 48 and the gear 44 are respectively provided concentrically.

Further, proximal end sides of the pulling wires 10r, 101, 10u and 10d are respectively wound around outer peripheries of the respective pulleys 42r, 421, 42u and 42d. Further, the pulley 42 transmits a pull assisting force from the friction plate 48 to the pulling wire 10 by contacting the friction plate 48 with a frictional force by input operation of the joystick 8.

Note that the pulley 42 rotates in the one direction R1 with the friction plate 48 after the pulley 42 contacts the friction plate 48, but does not rotate integrally with the friction plate 48, and rotates in the one direction R1 while sliding on the friction plate 48.

Further, for example, the pulley 42r contacts the friction plate 48r by the joystick 8 being tilted in the right direction, and at this time, the pulleys 421, 42d and 42u do not contact the friction plates 481, 48d and 48u.

However, when the joystick 8 is tilted to a position between the up direction and the right direction, the pulley 42u also contacts the friction plate 48u, similarly as in the first and the second embodiments described above. Namely, when the joystick 8 is tilted in the intermediate directions of up, down, left and right, two of the pulleys contact two of the friction plates.

Furthermore, the pulley 42 is configured by a material with a higher modulus of elasticity than the friction plate 48. Namely, the friction plate 48 is formed to be softer than the pulley 42.

Therefore, in the present embodiment, the pulley 42 configures one member, and the friction plate 48 configures the other member.

Note that in the present embodiment, the proximal end sides of the pulling wires 10r, 101, 10u and 10d are not connected to the joystick 8, but are only simply wound around the outer peripheries of the respective pulleys 42r, 421, 42u and 42d.

As shown in Fig. 14, the joystick 8 has the suspension frame 8t with the shape in plan view being cruciform at the bottom portion at the operation portion 5 side. The joystick 8 causes the respective pulleys 42r, 42l, 42u and 42d to contact the respective friction plates 48r, 481, 48u and 48d with a frictional force by pressing down the respective pulleys 42r, 421, 42u and 42d along the axial direction J as shown in Fig. 15 by using the respective end portions 8r, 81, 8u and 8d of the suspension frame 8t.

Note that in Fig. 15 and Fig. 16, only the mechanism relating to the pulley 42r is shown in order to simplify the drawing, and illustration of mechanisms relating to the pulleys 421, 42u and 42d is omitted.

The motor 46 and the gear 45 cause the friction plates 48r, 481, 48d and 48u to rotate in the one direction R1 via the gears 44r, 441, 44d and 44u. Note that the motor 46 is configured to rotate in the one direction R1 at all times when the power supply of the endoscope 1 is on.

Further, on a bottom surface 42rt which is a surface of the pulley 42r that contacts the friction plate 48r, a concave groove 42rh with a shape in plan view being cruciform is formed as shown in Fig. 16.

Further, in the present embodiment, the concave groove 42rh configures a removal portion that removes the wear debris M that is scraped from the friction plate 48r and adheres to the friction plate 48r by the pulley 42r contacting the friction plate 48r, from the friction plate 48r.

Note that concave grooves are also formed in bottom surfaces of the pulleys 421, 42d and 42u.

Next, an operation of the present embodiment will be described.

When the joystick 8 is not operated, the pulley 42 is not in contact with the friction plate 48 as shown in Fig. 15.

In order to cause the bending portion 4 to bend by using the aforementioned configuration in the above state, the joystick 8 is tilted, for example, in the right direction first, whereby the pulley 42r moves along the shaft 43r by being pressed along the axial direction J and contacts the friction plate 48r with a frictional force.

After the pulley 42r contacts the friction plate 48r, the pulley 42r rotates in the one direction R1 with the friction plate 48r. Thereby, the pull assisting force from the friction plate 48r is transmitted to the pulling wire 10r, and thereby the pulling wire 10r is pulled, whereby the bending portion 4 to which the distal end of the pulling wire 10r is fixed bends in the right direction.

Note that when the input operation of the joystick 8 is released, the pulley 42r separates from the friction plate 48r, and therefore, the pulling wire 10r is slackened, whereby the bending portion 4 returns to an unbending state.

Note that the above similarly applies to the occasion in which the bending portion 4 is caused to bend in the down direction, the right direction and the left direction.

Here, when the pulley 42r contacts the friction plate 48r with a frictional force, the friction plate 48r is scraped by the pulley 42r with contact, because the friction plate 48r is formed to be softer than the pulley 42r as described above.

Note that the wear debris M that is scraped adheres to the friction plate 48r that is softer than the pulley 42r as shown in Fig. 12 for the aforementioned reason, but the wear debris M that adheres to the friction plate 48r is peeled and removed from the friction plate 48r by the concave groove 42rh that is formed in the bottom surface 42rt of the pulley 42r with rotation in the one direction R1.

Note that removal of the wear debris M in the concave groove 42rh can be performed in a state in which the pulley 42r is in contact with the friction plate 48r, and in the case in which the pulley 42r rotates in the one direction R1 while the pulley 42r is in contact with the friction plate 48r and sliding.

Note that the above similarly applies to the occasion in which the bending portion 4 is caused to bend in the down direction, the right direction and the left direction.

As above, in the present embodiment, it is shown that the friction plate 48 is formed to be softer than the pulley 42. Further, it is shown that the wear debris M that is scraped from the friction plate 48 and adheres to the friction plate 48 with contact of the pulley 42 to the friction plate 48 is peeled and removed from the friction plate 48 by the concave groove that is formed in the bottom surface of the pulley 42.

According to the above, the wear debris M does not enter the space between the friction plate 48 and the pulley 42, and therefore, it does not happen that when the pulley 42 contacts the friction plate 48 with a frictional force, the aforementioned pull assisting force does not become constant and becomes unstable in addition to that vibration and unusual sound occur due to the wear debris M.

From the above, the endoscope 1 can be provided, which has the configuration that can easily and simply remove the wear debris M that is generated with contact of the pulley 42 to the friction plate 48, and adheres to the friction plate 48.

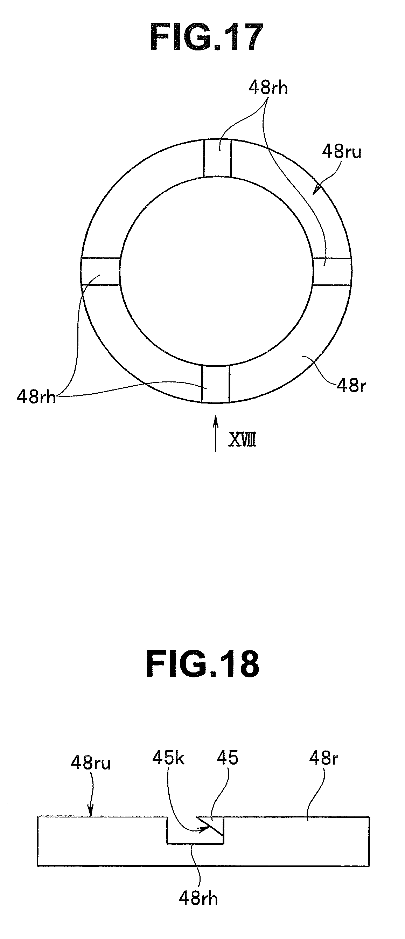

Note that hereinafter, modifications will be shown with use of Fig. 17 and Fig. 18. Fig. 17 is a plan view showing the modification in which a concave groove is not formed in the bottom surface of the pulley of Fig. 15, but a concave groove is formed in a surface of the friction plate that contacts the pulley. Fig. 18 is a side view showing the modification in which a cleaning blade is provided in the concave groove of Fig. 17, from a XVII direction in Fig. 17.

In the aforementioned present embodiment, the pulley 42 is configured by the material with a higher modulus of elasticity than the friction plate 48. Namely, it is shown that the friction plate 48 is formed to be softer than the pulley 42.

The friction plate 48 is not limited to the above, and the friction plate 48 is configured by a material with a higher modulus of elasticity than the pulley 42, totally contrarily to the present embodiment. Namely, the pulley 42 may be formed to be softer than the friction plate 48. In this case, the friction plate 48 configures the one member, and the pulley 42 configures the other member.

In the configuration as above, with contact of the pulley 42r to the friction plate 48r, the wear debris M generated by the pulley 42r being scraped adheres to the pulley 42r that is softer than the friction plate 48r as described above with contact of the pulley 42r to the friction plate 48r, but the wear debris M that adheres to the bottom surface 42rt of the pulley 42r is removed by the concave groove 48rh with the shape in plan view being cruciform that is formed in a top surface 48ru that is a surface of the friction plate 48r that contacts the pulley 42r, with rotation of the pulley 42r, as shown in Fig. 17.

Therefore, in the configuration in which the pulley 42r is formed to be softer than the friction plate 48r, the concave groove 48rh configures a removal portion that removes the wear debris M that is scraped from the pulley 42r and adheres to the bottom surface 42rt by the pulley 42r contacting the friction plate 48r from the bottom surface 42rt.

Further, as shown in Fig. 18, the concave groove 48rh may be provided with a cleaning blade 46 that has an inclined surface 45k for positively removing the wear debris M from the bottom surface 42rt.

Note that the above also applies to mechanisms that cause the bending portion 4 to bend in the down direction, the right direction and the left direction.

In the configuration as above, the endoscope 1 can be provided, which has the configuration that can easily and simply remove the wear debris M that is generated with contact of the pulley 42 to the friction plate 48 and adheres to the pulley 42.

Note that in the first to the third embodiments described above, the operation member is shown with the joystick 8 cited as an example, but the operation member is not limited thereto, and may be a knob, a trackball, a slide pad or the like as a matter of course.

Further, in the first to the third embodiments described above, the configuration that causes the bending portion of the insertion portion of the endoscope to bend is shown by being cited as an example, but the present invention is not limited thereto, and is also applicable to the configurations that cause bending portions of other insertion apparatuses such as a guide tube, various treatment instruments that do not have observation means, and a manipulator.

Furthermore, in the first to the third embodiments described above, the action portion is shown with the bending portion 4 cited as an example, but the action portion is not limited thereto, and may be anything if only it is operated by pulling of a pulling member, such as a known treatment instrument raising table.

The present application is filed claiming the priority of Japanese Patent Application No. 2012-139939 filed in Japan on June 21, 2012, and the above described disclosure is incorporated by reference in the present description, claims and drawings.

## Claims

1. An insertion apparatus which includes an insertion portion to be inserted into a subject and in which an action portion is provided at the insertion portion, the insertion apparatus comprising:
a pulling member that is inserted through the insertion portion, has a distal end in an insertion direction of the insertion portion fixed to the action portion, and causes the action portion to act by pulling;
an operation member that is provided at an operation portion at a proximal end in the insertion direction of the insertion portion for performing an input of a pulling operation of the pulling member;
a drive force generating member that is provided in the operation portion and capable of providing a pull assisting force to the pulling member by rotation;
a drive force assisting member that is provided in the operation portion and contacts the drive force generating member with a friction force with an input operation of the operation member and thereby transmits the rotation of the drive force generating member to the pulling member; and
a removal portion that is provided at any one member having a higher modulus of elasticity out of the drive force generating member and the drive force assisting member, and removes wear debris that is scraped from the other member having a low modulus of elasticity with contact of the drive force assisting member to the drive force generating member from the other member.

2. The insertion apparatus according to claim 1,
wherein the removal portion is formed into a blade shape having an inclined surface or formed into a concave shape at a position facing the other member having the low modulus of elasticity, in the one member having the higher modulus of elasticity.

3. The insertion apparatus according to claim 1 or 2,
wherein the other member having the low modulus of elasticity comprises an inner layer portion, and an outer layer portion that is provided on an outer side of the inner layer portion and contacts the one member, and
the outer layer portion is configured by a material having a lower modulus of elasticity than the inner layer portion.

4. The insertion apparatus according to any one of claims 1-3,
wherein a proximal end of the pulling member in the insertion direction is connected to the operation member, the drive force generating member is a pulley that rotates in one direction, and the drive force assisting member is a C-shaped ring that is contactable with an outer periphery of the pulley, has an outer periphery around which an intermediate position of the pulling member in the insertion direction is wound, and is partially cut out,
the C-shaped ring is reduced in diameter with pulling of the pulling member by the input operation of the operation member, contacts the pulley with the friction force and rotates in the one direction with the pulley, and thereby transmits the pull assisting force from the pulley to the pulling member.

5. The insertion apparatus according to claim 4,
wherein the C-shaped ring is configured by a material having a higher modulus of elasticity than the pulley, and
a cutout portion of the C-shaped ring configures the removal portion.

6. The insertion apparatus according to claim 4,
wherein the pulley is configured by a material having a higher modulus of elasticity than the C-shaped ring, and
a concave portion that is formed in the outer periphery of the pulley configures the removal portion.

7. The insertion apparatus according to any one of claims 1-3,
wherein the drive force generating member is a pulley that rotates in one direction, and the drive assisting member is configured by a pressing member rotatable by the input operation of the operation member, and a pressed member that has an intermediate position of the pulling member in the insertion direction locked thereto, and contacts the pulley with a frictional force by pressing by rotation of the pressing member, and
the pressed member is pressed by the pressing member by the input operation of the operation member and contacts an outer periphery of the pulley with the friction force, and thereby transmits the pull assisting force from the pulley to the pulling member.

8. The insertion apparatus according to claim 7,
wherein the pressed member is configured by a material with a higher modulus of elasticity than the pulley, and
the pressed member is provided with the removal portion.

9. The insertion apparatus according to claim 7,
wherein the pulley is configured by a material having a higher modulus of elasticity than the pressed member, and
a concave portion that is formed in the pulley configures the removal portion.

10. The insertion apparatus according to any one of claims 1-3,
wherein the drive force generating member is a friction plate that rotates in one direction, and the drive assisting member is a pulley around which a proximal side of the pulling member in the insertion direction is wound and that contacts the friction plate with a frictional force by the input operation of the operation member,
the pulley contacts the friction plate with the friction force by the input operation of the operation member and thereby transmits the pull assisting force from the friction plate to the pulling member.

11. The insertion apparatus according to claim 10,
wherein the pulley is configured by a material with a higher modulus of elasticity than a modulus of elasticity of the friction plate, and
a groove in a concave shape that is formed in a surface that contacts the friction plate in the pulley configures the removal portion.

12. The insertion apparatus according to claim 10,
wherein the friction plate is configured by a material with a higher modulus of elasticity than a modulus of elasticity of the pulley, and
a groove in a concave shape that is formed in a surface that contacts the pulley in the friction plate configures the removal portion.
